(19) Europäisches Patentamt
European Patent Office
Office européen des brevets

(11) **EP 3 092 940 A1**

(12) **EUROPEAN PATENT APPLICATION**
published in accordance with Art. 153(4) EPC

(43) Date of publication:
**16.11.2016 Bulletin 2016/46**

(51) Int Cl.:
**A61B 1/00** (2006.01)   **G02B 23/26** (2006.01)

(21) Application number: **15789296.9**

(86) International application number:
**PCT/JP2015/059324**

(22) Date of filing: **26.03.2015**

(87) International publication number:
**WO 2015/170525 (12.11.2015 Gazette 2015/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **07.05.2014 JP 2014096290**

(71) Applicant: **Olympus Corporation Tokyo 192-8507 (JP)**

(72) Inventor: **ONOE, Tomomichi Hachioji-shi, Tokyo 192-8507 (JP)**

(74) Representative: **Gunzelmann, Rainer Wuesthoff & Wuesthoff Patentanwälte PartG mbB Schweigerstraße 2 81541 München (DE)**

(54) **ENDOSCOPE**

(57) An endoscope (2) includes an observation optical system (41), two illumination optical systems (42, 43), two inclined plane portions DS11 and DS12 provided between a peripheral edge portion of an objective lens at a distal end of the observation optical system and peripheral edge portions of two illumination lenses, and two edge line portions AL1 and AL2 formed at intermediate portions of the two inclined plane portions DS11 and DS12, respectively, to protrude in a distal end direction of the insertion portion 12, between the peripheral edge portion of the objective lens and the peripheral edge portion of the two illumination lenses, wherein a height H0 of an incident surface IS of the objective lens of the observation optical system is higher than a height HBi of the two edge line portions AL1 and AL2, and exit surfaces ES of the two illumination optical systems are lower than the height HBi of the two edge line portions AL1 and AL2. Further, the two illumination lenses are placed to be point-symmetric with respect to a center axis of the objective lens when a distal end portion (17) is seen from a distal end direction.

**FIG. 6**

**Description**

Technical Field

The present invention relates to an endoscope, and particularly relates to an endoscope that emits illuminating lights from at least two illumination optical systems provided in a distal end portion of an insertion portion.

Background Art

Conventionally, endoscopes have been widely used in a medical field and an industrial field. An endoscope is configured so that illuminating lights are emitted from one or more illuminating windows provided at a distal end portion of an insertion portion, and reflection lights from an object illuminated with the illuminating lights are incident on an objective lens of an observation window.

When the light emitted from the illuminating window directly enters the objective lens, a flare occurs to an endoscopic image. Consequently, Japanese Patent Application Laid-Open Publication No. 2007-289355 proposes an endoscope in which an inclined plane to be a light shielding wall is provided between an illuminating window and an objective lens so that the light emitted from the illuminating window does not directly enter the objective lens.

However, in the endoscope according to the above described proposal, consideration is not given to the problem of illumination unevenness of illuminating lights which are respectively emitted from two illuminating windows. The illuminating lights that are respectively emitted from the two illuminating windows are the brightest in optical axis center portions of the illuminating lights, and light amounts decrease toward peripheries from centers.

Consequently, when the illuminating lights which are respectively emitted from the two illuminating windows are used, a dark portion which the illuminating lights do not sufficiently fall on occurs between two illumination ranges by the illuminating lights. That is, light distribution unevenness occurs. Light distribution unevenness appears as unevenness in brightness in the image of an object. In particular, at the time of proximity observation, the illumination ranges of respective illuminating lights are narrow, and therefore, unevenness in the brightness of an image becomes remarkable.

Therefore, the present invention has an object to provide an endoscope in which light distribution unevenness by two illuminating lights that are respectively emitted from two illuminating windows is reduced.

Disclosure of Invention

Means for Solving the Problem

An endoscope of one aspect of the present invention includes an observation optical system that is placed in a distal end portion of an insertion portion, and is capable of enlarged observation optically, a first illumination optical system that is placed in the distal end portion, has a first illumination lens in a distal end, and emits a first illuminating light, a second illumination optical system that is placed in the distal end portion, has a second illumination lens in a distal end, and emits a second illuminating light, a first inclined plane portion that is provided between a peripheral edge portion of an objective lens in a distal end of the observation optical system and a peripheral edge portion of the first illumination lens, a second inclined plane portion that is provided between a peripheral edge portion of the objective lens and a peripheral edge portion of the second illumination lens, a first edge line portion that is formed at an intermediate portion of the first inclined plane portion, and is formed to protrude in a distal end direction of the insertion portion, between the peripheral edge portion of the objective lens and the peripheral edge portion of the first illumination lens, and a second edge line portion that is formed at an intermediate portion of the second inclined plane portion, and is formed to protrude in the distal end direction of the insertion portion, between the peripheral edge portion of the objective lens and the peripheral edge portion of the second illumination lens, wherein the observation optical system, the first illumination optical system and the second illumination optical system are placed so that relations of H0 > HB1 > HL1, and H0 > HB2 > HL2 are established, when a height to an incident surface of the objective lens of the observation optical system from a predetermined position in the distal end direction of the insertion portion is set as H0, a height to the first edge line portion from the predetermined position in the distal end direction of the insertion portion is set as HB1, a height to the second edge line portion from the predetermined position in the distal end direction of the insertion portion is set as HB2, a height to an exit surface of the first illumination optical system from the predetermined position in the distal end direction of the insertion portion is set as HL1, and a height to an exit surface of the second illumination optical system from the predetermined position in the distal end direction of the insertion portion is set as HL2, and the first illumination lens and the second illumination lens are placed to be point-symmetric or substantially point-symmetric with respect to a center axis of the objective lens of the observation optical system when the distal end portion is seen from the distal end direction, or to be line-symmetric or substantially line-symmetric with respect to a line passing through the center axis of the objective lens of the observation optical system.

Brief Description of the Drawings

Fig. 1 is a configuration diagram showing a configuration of an endoscope apparatus according to an embodiment of the present invention;

Fig. 2 is a front view of a distal end face of a distal end portion 17 according to the embodiment of the present invention;

Fig. 3 is a sectional view of a distal end portion of the distal end portion 17 along a line III-III in Fig. 2;

Fig. 4 is a sectional view of the distal end portion of the distal end portion 17 along a line IV-IV in Fig. 2;

Fig. 5 is a front view of a distal end face 17a of the distal end portion 17 for explaining an inclined plane portion formed in the distal end face 17a of the distal end portion 17, according to the embodiment of the present invention;

Fig. 6 is a partial sectional view for explaining configurations of a distal end portion of an image pickup unit 41 in the distal end portion 17, and a distal end portion of a light guide unit 42, according to the embodiment of the present invention;

Fig. 7 is a view showing illuminating lights from illuminating windows 22 and 23, and an observation range of an observation window 21, according to the embodiment of the present invention;

Fig. 8 is a view showing an illuminating light from an illuminating window 24, and the observation range of the observation window 21, according to the embodiment of the present invention;

Fig. 9 is a view for explaining a light shielding effect of an edge line portion AL formed in a distal end cover 32, at a time of proximity observation by an endoscope, according to the embodiment of the present invention; and

Fig. 10 is a view for explaining a range of a field of view of an image pickup device 41c, with respect to dispositions of the illuminating windows 22 and 23, according to the embodiment of the present invention.

Best Mode for Carrying Out the Invention

Hereinafter, an embodiment of the present invention will be described with reference to the drawings.

(Configuration of endoscope apparatus)

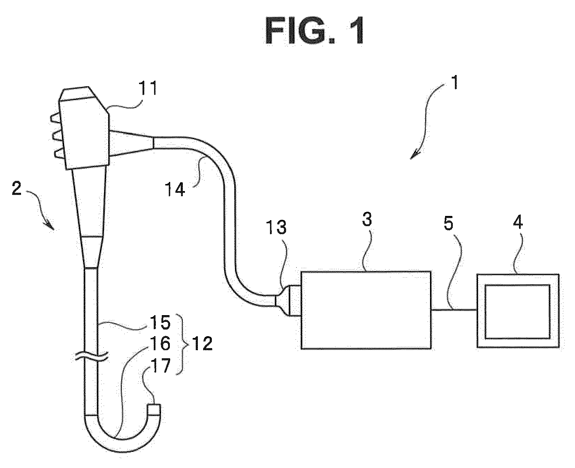

Fig. 1 is a configuration diagram showing a configuration of an endoscope apparatus according to the present embodiment. An endoscope apparatus 1 includes an endoscope 2, a main body apparatus 3 and a monitor 4. The monitor 4 is connected to the main body apparatus 3 by a cable 5.

The endoscope 2 is an electronic endoscope in this case, and includes an operation portion 11 including a knob and a switch for performing a bending operation and control of various conduits, an insertion portion 12 that has a proximal end portion connected to the operation portion 11 and is inserted into a body cavity, and a universal cord 14 that is extended from the operation portion 11 and has a connector portion 13 at a distal end portion. The connector portion 13 is connected to the main body apparatus 3.

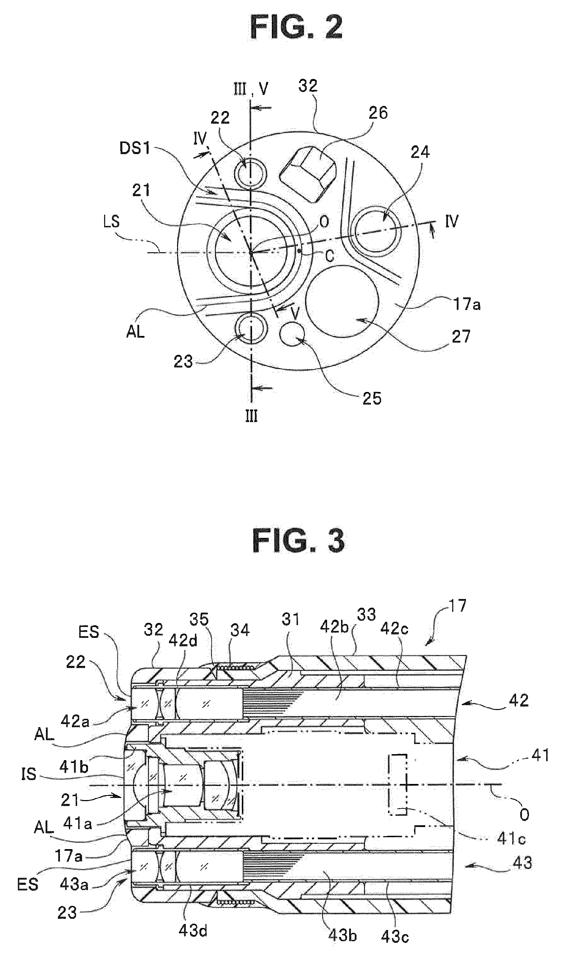

The insertion portion 12 has a flexible tube portion 15 having flexibility, a bending portion 16 that is provided at a distal end side of the flexible tube portion 15, and a distal end portion 17 that is provided at a distal end side of the bending portion 16. The distal end portion 17 is a distal end rigid portion, and in a distal end face of the distal end portion 17, illuminating windows, an observation window and the like that will be described later are provided. A distal end portion of a light guide that guides illuminating lights is placed inside the distal end portion 17, and illuminating lights are emitted from three illuminating windows on the distal end face of the distal end portion 17. Further, inside of the distal end portion 17, an image pickup device 41c (refer to Fig. 3) such as a CMOS image sensor is placed, and receives reflection light from an object that receives light through the observation window to subject the reflection light to photoelectric conversion to generate an image pickup signal.

The main body apparatus 3 contains a light source apparatus and a processor. The light source apparatus has a light emitting portion such as an LED, and supplies light to a proximal end portion of the light guide which is inserted through the insertion portion 12. The processor controls an operation of the entire endoscope apparatus 1, receives an image pickup signal from the image pickup device in the distal end portion 17, generates an endoscopic image, outputs the endoscopic image to the monitor 4 via the cable 5, and records the endoscopic image in a storage apparatus not illustrated.

Further, an air feeding source, a water feeding source and a suction source for performing air feeding, water feeding, suction and the like to various conduits contained in the endoscope 2 are provided in the main body apparatus 3, or an apparatus not illustrated that is further connected to the main body apparatus 3.

(Configuration of distal end portion of insertion portion 12)

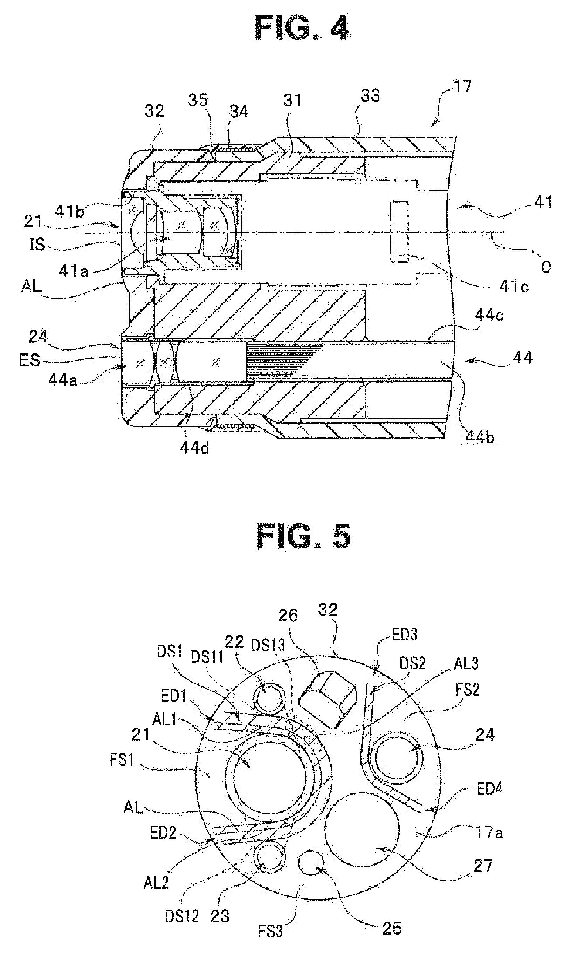

Fig. 2 is a front view of the distal end face of the distal end portion 17. Fig. 3 is a sectional view of a distal end portion of the distal end portion 17 along a line III-III in Fig. 2. Fig. 4 is a sectional view of the distal end portion of the distal end portion 17 along a line IV-IV in Fig. 2.

As shown in Fig. 2, in a surface (hereinafter, referred to as a distal end face) 17a at a distal end side of the distal end portion 17 of the insertion portion 12 of the endoscope 2 of the present embodiment, an observation window 21, three illuminating windows 22, 23 and 24, a forward water feeding opening 25, an air/water feeding nozzle 26 and a treatment instrument opening 27 are provided, and disposed.

A center axis O of the observation window 21 does not correspond to a center axis C of the distal end portion 17. The observation window 21 is disposed by being deviated from the center axis C by a predetermined amount.

The observation window 21 is an end face (hereinafter, an objective face) at an observation target side in an image pickup optical system of an image pickup unit 41 which will be described later. Further, the illuminating windows 22, 23 and 24 are respectively end faces (hereinafter, objective faces) at the observation target side in illumination optical systems of light guide units 42, 43 and 44 that will be described later.

When the distal end face 17a of the distal end portion 17 is viewed directly from a front, that is, when the distal end face 17a is viewed from a distal end direction, the illuminating windows 22 and 23 are disposed at positions which are point-symmetric or positions which are substantially point-symmetric with respect to a point of the center axis O of the observation window 21, or at positions which are line-symmetric or positions which are substantially line-symmetric with respect to a line LS (shown by a chain double-dashed line in Fig. 2) which passes through the center axis O.

Here, the illuminating windows 22 and 23 are disposed so that distances from an observation optical system of the observation window 21 are equal to each other, in the distal end portion 17, whereas the illuminating window 24 is placed at a position which is farther than the illuminating windows 22 and 23, in the distal end portion 17.

As shown in Fig. 3, a distal end rigid member 31 which is in a columnar shape and made of a metal such as stainless steel is provided in the distal end portion 17. In the distal end rigid member 31, a plurality of holes are formed, which are for fixing various members corresponding to the observation window 21, the three illuminating windows 22, 23 and 24, the forward water feeding opening 25, the air/water feeding nozzle 26 and the treatment instrument opening 27 which are disposed in the distal end face of the distal end portion 17.

A distal end cover 32 of a resin is put on a distal end side of the distal end rigid member 31. In the distal end cover 32, a plurality of holes are also formed in positions corresponding to the plurality of holes which are formed in the distal end rigid member 31. An outer sheath 33 is put on the distal end portion 17 to the flexible tube portion 15. A distal end portion of the outer sheath 33 is fixed to the distal end rigid member 31 by a thread 34 being wound on the distal end portion, and an adhesive 35 being further applied to the distal end portion.

As shown in Fig. 3, the image pickup unit 41 is formed at a rear side from the observation window 21. Further, the light guide units 42, 43 and 44 are formed respectively at rear sides of the three illuminating windows 22, 23 and 24. A forward water feeding conduit (not illustrated) is placed at a rear side of the forward water feeding opening 25. An air/water feeding conduit (not illustrated) is placed at a rear side of the air/water feeding nozzle 26. The treatment instrument opening 27 communicates with a treatment instrument insertion channel (not illustrated).

With use of Fig. 3 and Fig. 4, configurations of the image pickup unit and the light guide units will be described.

The image pickup unit 41 has a frame 41b including an objective lens 41a at a distal end side. Further, the image pickup unit 41 has the image pickup device 41c such as a CMOS image sensor, which receives light from an object, which passes through the objective lens 41a, subjects the light to photoelectric conversion, and outputs an image pickup signal. A distal end portion of the image pickup unit 41 is fitted into a predetermined hole of the distal end rigid member 31 and is fixed by an adhesive.

An observation optical system of the image pickup unit 41 is an optical system capable of enlarged observation optically. That is, the observation optical system including the objective lens 41a is an observation optical system which is placed in the distal end portion 17 of the insertion portion 12 and is capable of enlarged observation optically.

As shown in Fig. 3, the light guide unit 42 is formed of an illumination lens 42a formed of a plurality of lenses, and an optical fiber bundle 42b which is a light guide. A distal end portion of the optical fiber bundle 42b is fixed into a metal pipe 42c by an adhesive or the like. The illumination lens 42a is inserted into a frame 42d to be fixed to the frame 42d, and the metal pipe 42c is internally inserted from a proximal end side of the frame 42d to be fixed to the frame 42d.

The light guide unit 43 is also formed of an illumination lens 43a formed of a plurality of lenses, and an optical fiber bundle 43b that is a light guide, similarly to the light guide unit 42. A distal end portion of the optical fiber bundle 43b is fixed into a metal pipe 43c by an adhesive or the like. The illumination lens 43a is inserted into a frame 43d to be fixed to the frame 43d, and the metal pipe 43c is internally inserted from a proximal end side of the frame 43d to be fixed to the frame 43d.

As shown in Fig. 4, the light guide unit 44 is also formed of an illumination lens 44a formed of a plurality of lenses, and an optical fiber bundle 44b that is a light guide, similarly to the light guide unit 42. A distal end portion of the optical fiber bundle 44b is fixed into a metal pipe 44c by an adhesive or the like. The illumination lens 44a is inserted into a frame 44d, and the metal pipe 44c is internally inserted from a proximal end side of the frame 44d to be fixed to the frame 44d.

Illumination optical systems of the light guide units 42, 43 and 44 are respectively placed in the distal end portion 17, respectively have illumination lenses and emit illuminating lights.

Respective distal end portions of the image pickup unit 41 and the light guide units 42, 43 and 44 are inserted into corresponding holes formed in the distal end cover 32, and are fixed by an adhesive.

Respective distal end faces of the light guide lenses 42, 43 and 44 are exit surfaces ES for illuminating lights, and a distal end face of the objective lens 41a is an incident surface IS for reflection lights from an object.

In the present embodiment, the additional illuminating window 24 is provided in addition to the illuminating windows 22 and 23, and the illuminating window 24 is an illuminating window for sufficiently ensuring a light amount of illuminating light at a non-proximal time.

(Heights and dispositions of observation window, three illuminating windows and inclined plane portion)

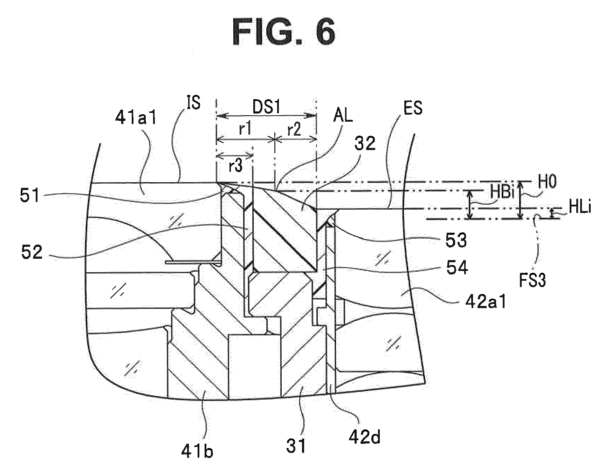

Fig. 5 is a front view of the distal end face 17a of the distal end portion 17 for explaining an inclined plane portion formed in the distal end face 17a of the distal end portion 17. Fig. 6 is a partial sectional view for explaining configurations of the distal end portion of the image pickup unit 41 in the distal end portion 17, and the distal end portion of the light guide unit 42.

An inclined plane portion DS1 is provided between the observation window 21 and the illuminating windows 22 and 23.

As shown in Fig. 5, the inclined plane portion DS1 shown by oblique lines is formed into a U-shape, around the observation window 21 on the distal end face 17a in the distal end cover 32. The inclined plane portion DS1 is formed to pass between the observation window 21 and the illuminating window 22 from one edge portion ED1 at the distal end face 17a in the distal end cover 32, surround a part of a peripheral portion of the observation window 21, and pass further between the observation window 21 and the illuminating window 23 to reach another edge portion ED2 at the distal end face 17a. A region surrounded by the two edge portions ED1 and ED2 and the observation window 21 is a flat face FS1.

As shown in Fig. 6, a concave lens 41a1 at the distal end of the objective lens 41a is fixed to the frame 41b by an adhesive 51. The frame 41b in the distal end portion of the image pickup unit 41 is fixed to the distal end cover 32 by an adhesive 52. Note that Fig. 6 is a partial sectional view of a part between the image pickup unit 41 and the light guide unit 42, a partial section of a part between the image pickup unit 41 and the light guide unit 43 are similar to Fig. 6. That is, a configuration of the part between the image pickup unit 41 and the light guide unit 43 is the same as the configuration shown in Fig. 6.

A distal end lens 42a1 of the illumination lens 42a is fixed to the frame 42d by an adhesive 53. The frame 42d in the distal end portion of the light guide unit 42 is fixed to the distal end cover 32 by an adhesive 54.

As shown in Fig. 6, the inclined plane portion DS1 more specifically has an inclined portion r1 that gradually inclines toward an exit surface ES from an end portion of the observation window 21, and an inclined portion r2 that has an inclination angle larger than an inclination angle of the inclined portion r1 toward an outer edge of the adhesive 54 that surrounds the exit surface ES from an outer edge of the inclined portion r1. A boundary between the inclined portions r1 and r2 is an edge line portion AL.

Further, a surface portion r3 of the adhesive 52 for fixing the frame 41b of the image pickup unit 41 to the distal end cover 32 is a part of the inclined portion r1. That is, the surface portion r3 at a distal end side of the adhesive 52 and the inclined portion r1 of the distal end cover 32 have a continuous surface.

Note that although the two inclined portions r1 and r2 are formed at a distal end face side of the distal end cover 32 here, only an inclined portion corresponding to a part of the inclined portion r2 may be formed in the distal end face of the distal end cover 32, and the inclined portion r1 including the edge line portion AL and a part of the inclined portion r2 may be formed in the surface portion r3 at the distal end side of the adhesive 52.

Accordingly, parts of the inclined portions r1 and r2 of the inclined plane portion DS1 are formed at the distal end cover 32 which is the distal end cover member which is put on the distal end portion 17, and a remaining portion of the inclined portion r1 is formed by the adhesive 52. Parts of the inclined portions r1 and r2 of the inclined plane portion DS1 may be formed by the adhesive 52 which connects and fixes the distal end cover 32 which is put on the distal end portion 17 and the image pickup unit 41 which is the observation optical system.

In the present embodiment, the two inclined portions r1 and r2 are formed at the distal end face side of the distal end cover 32, because a shape of the edge line portion AL which shields an illuminating light emitted from the exit surface ES is easily formed more accurately by resin molding.

As shown in Fig. 6, a peripheral portion of the exit surface ES is provided with the adhesive 54 for fixing the frame 42d of the light guide unit 42 to the distal end cover 32, and a distal end side face of the adhesive 54 and the exit surface ES are on a same plane.

That is, the inclined portion r1 includes the surface portion r3 at the distal end face side of the adhesive 52, and the inclined portion r2 is formed at the distal end face 17a of the distal end portion 17 so that the inclination angle becomes large from the inclined portion r1 to the inclined portion r2. An outer edge portion of the inclined portion r2 is at a same height as the exit surface ES.

Further, as shown in Fig. 5, an inclined plane portion DS2 shown by oblique lines is also formed around the illuminating window 24 at the distal end face 17a in the distal end cover 32.

The inclined plane portion DS2 is formed to pass between the air/water feeding nozzle 26 and the illuminating window 24 from one edge portion ED3 at the distal end face 17a in the distal end cover 32, surround a part of a peripheral portion of the illuminating window 24, pass further between the treatment instrument opening 27 and the illuminating window 24 to reach another edge portion ED4 at the distal end face 17a. A region surrounded by the two edge portions ED3 and ED4 and the illuminating window 24 is a flat surface FS2.

A region between the inclined plane portions DS1 and DS2 is a flat surface FS3. Accordingly, the distal end face 17a is not flat throughout an entire face.

Next, heights of the respective portions will be described in detail.

The inclined plane portion DS1 which is formed between the exit surface ES of the illumination lens 42a and the incident surface IS of the objective lens 41a has the edge line portion AL. Further, on the distal end face 17a, the two exit surfaces ES and the incident surface IS differ from each other in height.

Heights (that is, distances in a distal end direction of the distal end portion 17 from the flat surface FS3) of the exit surface ES, the edge line portion AL and the incident surface IS with a position of the flat surface FS3 as a predetermined position in a distal end direction of the insertion portion 12 being set as a reference will be compared next.

Here, the two exit surface ES and the flat surface FS3 are on the same plane. That is, the two exit surfaces ES of the illuminating windows 22 and 23 and the flat surface FS3 are on the same plane. Consequently, the distance to the exit surface ES from the position of the flat surface FS3 is zero. Note that in Fig. 6, in order to express that the flat surface FS3 and the two exit surfaces ES are functionally different surfaces, the flat surface FS3 and the two exit surfaces ES are shown as different from each other by the heights being caused to differ.

The distance to the exit surface ES from the flat surface FS3 is actually zero, and therefore when the heights from the flat surface FS3 of the insertion portion 12 to the respective exit surfaces ES of the two illumination optical systems are set as HL1 and HL2, a relation of

$$\mathrm{HL1 = HL2} \; (= 0) \qquad\qquad \dots (1)$$

is established.

Note that in Fig. 6, the heights of the two exit surfaces ES are denoted by HLi (here, i is 1 or 2).

Further, when the height of the edge line portion AL from the flat surface FS3 of the insertion portion 12 is set as HBi, a relation of

$$\mathrm{HBi > HL1 = HL2} \qquad\qquad \dots (2)$$

is established.

Further, when the height to the incident surface IS from the flat surface FS3 of the insertion portion 12 is set as H0, a relation of

$$\mathrm{H0 > HBi} \qquad\qquad \dots (3)$$

is established.

The exit surfaces ES, the edge line portion AL and the incident surface IS are disposed or formed so that the relations of expressions (1) to (3) are established.

That is, the observation optical system and the two illumination optical systems are placed in the distal end portion 17 so that a relation of

$$\mathrm{H0 > HBi > HL1 = HL2} \qquad\qquad \dots (4)$$

is established, when the height of the incident surface IS in the objective lens 41a of the observation optical system to a predetermined position (here, the position of the flat surface FS3) of the distal end portion 17 is set as H0, the height of the edge line portion AL is set as HBi, and the heights of the respective exit surfaces ES of the two illumination optical systems are set as HL1 and H12.

In other words, the height H0 is a distance from the flat surface FS3 to the incident surface IS of the objective lens 41 a, the height HBi is a distance from the flat surface FS3 to the edge line portion AL, and the heights HL1 and NL2 are respectively distances to the exit surfaces ES of the two illumination optical systems.

Note that here, the height HBi of the edge line portion AL is equal in the inclined plane portion DS1, but may differ in the inclined plane portion DS1. In Fig. 5, a height HB1 of an edge line portion AL1 at a portion DS 11 (shown by a dotted-line ellipse) between the observation window 21 and the illuminating window 22 in the inclined plane portion DS1, and a height HB2 of an edge line portion AL2 of a portion DS12 (shown by a dotted-line ellipse) between the observation window 21 and the illuminating window 23 in the inclined surface portion DS1 may be different from each other. In this case, the heights H0, HB1, HB2, HL1 and HL2 have relations that satisfy the following expressions.

$$H0 > HB1 > HL1 \qquad \qquad ... (5)$$

$$H0 > HB2 > HL2 \qquad \qquad ... (6)$$

The portion DS 11 is a portion in a range (a range shown by a dotted line) in which illuminating light advances from the illuminating window 22 to the observation window 21. The portion DS 12 is a portion in a range (a range shown by a dotted line) in which illuminating light advances from the illuminating window 23 to the observation window 21.

Further, in Fig. 5, a portion DS13 (shown by a dotted-line ellipse) in the inclined plane portion DS1 is an inclined plane portion which is provided between a peripheral edge portion of the objective lens 41 a and the air/water feeding nozzle 26. Consequently, an edge line portion AL3 that is formed to protrude in the distal end direction of the insertion portion 12 is also provided between the peripheral edge portion of the objective lens 41a and the air/water feeding nozzle 26, at an intermediate portion in the portion DS13.

In other words, the inclined plane portions of the three portions DS11, DS12 and DS 13 are included in the inclined plane portion DS1 which is a continuous inclined plane portion, and the inclined plane portion DS1 is formed to connect the two edge portions ED1 and ED2 of the distal end face 17a of the distal end portion 17 and surround a part of the peripheral edge portion of the objective lens 41 a.

The illuminating window 24 is located at a position more apart from the observation window 21 than the illuminating windows 22 and 23, and the exit surface ES of the illuminating window 24 and the incident surface IS of the observation window 21 are on substantially the same plane. A first reason of this is that since the illuminating window 24 is apart from the observation window 21, emission light from the illuminating window 24 cannot be sometimes shielded by the edge line portion AL. A second reason is that since the range of a field of view is narrow at a time of proximity photographing, an influence of light distribution unevenness by the illuminating window 24 which is apart from the observation window 21 is small. Further, a third reason is to enable illuminating light to reach a distant spot at a time of non-proximal photographing.

(Operation)

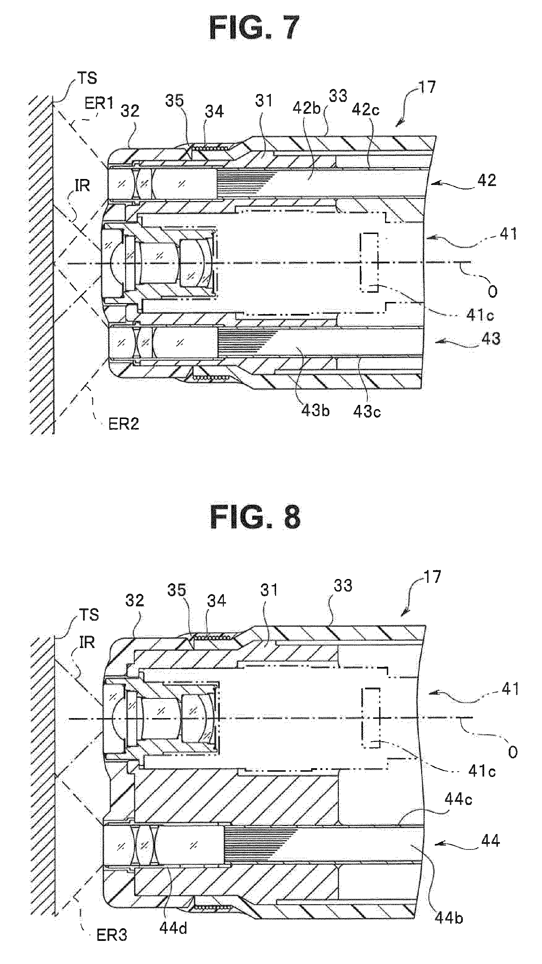

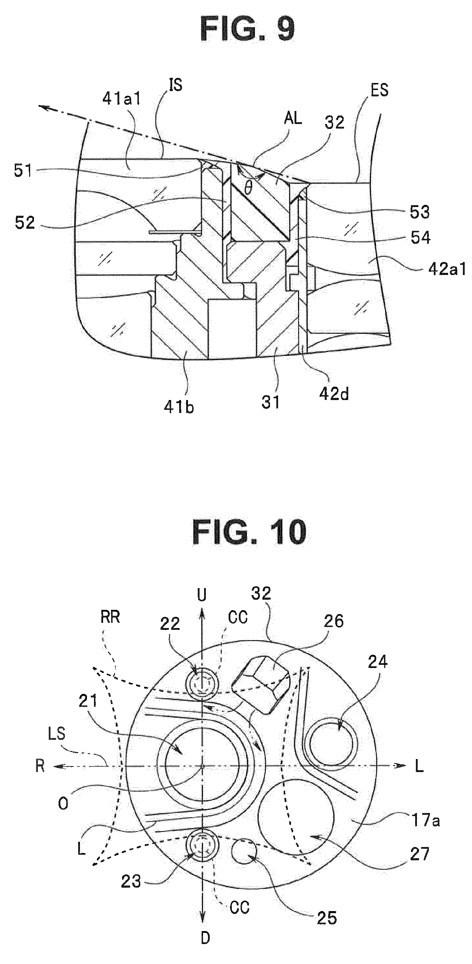

Fig. 7 is a view showing illuminating lights from the illuminating windows 22 and 23, and an observation range of the observation window 21. Fig. 8 is a view showing illuminating lights from the illuminating window 24, and the observation range of the observation window 21. Fig. 9 is a view for explaining a light-shielding effect of the edge line portion AL which is formed at the distal end cover 32, at a time of proximity observation by the endoscope.

As shown in Fig. 7, most of the illuminating lights emitted from the illuminating windows 22 and 23 are respectively emitted to illumination ranges ER1 and ER2 (shown by dotted lines) in the distal end direction of the distal end portion 17, and illuminate a living tissue TS which is an object. As shown in Fig. 8, most of illuminating lights emitted from the illuminating window 24 are emitted to an illumination range ER3 (shown by dotted lines) in the distal end direction of the distal end portion 17, and illuminate the living tissue TS which is an object. Reflection light from living tissue TS of the illuminating light is incident on the observation window 21 in a range IR of a field of view shown by an alternate long and short dash line.

Since the two exit surfaces ES are located at a proximal end side from the incident surface IS, the respective illumination ranges are enlarged, and even at the time of proximity observation, that is, at the time of enlarged observation, a portion in which the light amount of the illuminating light is small hardly occurs between the two illumination ranges. Consequently, light distribution unevenness by the two illuminating lights from the illuminating windows 22 and 23 decreases.

Furthermore, of the illuminating lights from the illuminating windows 22 and 23, the light which advances toward the observation window 21 is shielded by the inclined plane portion DS1 having the edge line portion AL. As shown in Fig. 9, of the illuminating lights which are emitted from the exit surfaces ES of the illuminating windows 22 and 23, the illuminating light in a range below the edge line portion AL (that is, a range lower than the above described height HBi) is shielded by the inclined plane portion DS1, and is not directly incident on the incident surface IS of the observation window 21.

As shown in Fig. 9, in each of the respective portions DS11 and DS12, an angle θ formed by the inclined portions r1 and r2 of the two inclined planes of the inclined plane portion DS1 which is divided by the edge line portion AL is an obtuse angle. Consequently, the observation window 21 protrudes in the distal end direction from the distal end face 17a, so that favorable cleaning performance for the observation window 21 by the air/water feeding nozzle 26 can be ensured, and favorable water draining performance on the observation window 21 can be also realized.

Since the two exit surfaces ES of the illuminating windows 22 and 23 which are placed to sandwich the observation window 21 are placed to be located at a proximal end side from the incident surface IS of the observation window 21, the illumination range of the illuminating light emitted from the illuminating window 22 and the illumination range of the illuminating light emitted from the illuminating window 23 are more enlarged on an object than when the exit surfaces ES of the illuminating windows 22 and 23 and the incident surface IS of the observation window 21 are on the same plane, a part between the two illumination ranges, that is, a vicinity of the center of an endoscopic image can be also irradiated with sufficient illuminating light, and light distribution unevenness of the two illuminating lights can be reduced.

Further, the inclined plane portion DS1 is provided, whereby the illuminating lights from the illuminating windows 22 and 23 are not directly incident on the observation window 21, and therefore, occurrence of a flare on the endoscopic image can be prevented.

Note that for the purpose of preventing occurrence of a flare, only the edge line portion AL1 of the portion DS11 and the edge line portion AL2 of the portion DS12 described above may be formed in the inclined plane portion DS1, as for the edge line portion AL. Consequently, the inclined plane portion DS1 can be provided at least only a range corresponding to the portion DS 11 and a range corresponding to the portion DS12.

That is, in the inclined plane portion DS1, the portion DS 11 of the inclined plane portion DS1 configures a first inclined plane portion which is provided between the peripheral edge portion of the concave lens 41a1 which is the objective lens at the distal end of the observation optical system and the peripheral edge portion of the illumination lens 42a1 at the distal end of the illumination optical system of the illuminating window 22. In the inclined plane portion DS1, the portion DS12 of the inclined plane portion DS1 configures a second inclined plane portion which is provided between the peripheral edge portion of the concave lens 41a1 which is the objective lens at the distal end of the observation optical system, and the peripheral edge portion of the illumination lens at the distal end of the illuminating optical system of the illuminating window 23.

The edge line portion AL1 in the portion DS 11 configures a first edge line portion which is formed at an intermediate portion of the first inclined plane portion, and is formed to protrude in the distal end direction of the insertion portion 12, between the peripheral edge portion of the concave lens 41a1 which is the objective lens at the distal end, and the peripheral edge portion of the illumination lens 42a1 of the illuminating window 22. The edge line portion AL2 in the portion DS12 configures a second edge line portion that is formed at an intermediate portion of the second inclined plane portion, and is formed to protrude in the distal end direction of the insertion portion 12, between the peripheral edge portion of the concave lens 41a1 which is the objective lens and the peripheral edge portion of the illumination lens of the illuminating window 23.

Further, while in the present embodiment, the third illuminating window 24 is provided on the distal end face 17a of the distal end portion 17, the illuminating window 24 is disposed in a position which is more apart from the observation window 21 than the illuminating windows 22 and 23, and the exit surface ES of the illuminating window 24 is placed at substantially the same height as the incident surface IS of the observation window 21.

That is, the third illumination optical system which has the illumination lens at the distal end, and emits illuminating light is placed in the distal end portion 17. The observation optical system and the third illumination optical system are placed so that when the height from the flat surface FS3 as the predetermined position to the exit surface ES of the third illumination optical system is set as HL3, a relation of

$$H0 = HL3 \qquad\qquad \dots (7)$$

is established. Consequently, a sufficient light amount of illuminating light can be ensured at the time of non-proximal observation without causing occurrence of a flare.

Furthermore, in the shape of the endoscopic image obtained by picking up the image by the image pickup device 41 c, vertical and lateral lengths are sometimes different. In general, a shape of an endoscopic image is a long rectangle which is long in a lateral direction. That is, in general, an endoscopic image has a shape in which a width in a vertical direction is shorter as compared with a height in a lateral direction. Therefore, the illuminating windows 22 and 23 are desirably disposed so that centers of the illumination ranges are located in the vertical direction of the obtained endoscopic image.

Fig. 10 is a view for explaining a range of a field of view of the image pickup device 41 c with respective to dispositions of the illuminating windows 22 and 23. In Fig. 10, U, D, L and R show respective directions of an up, a down, a left and a right of the endoscopic image which is obtained.

Reflection light that is incident through the observation window 21 is incident on the image pickup device 41c through the objective optical system. Here, as shown in Fig. 10, the range RR of the field of view of the image pickup device 41c is a bobbin type which extends laterally by an aberration of a lens. The range RR of the field of view differs in accordance with an angle around a center axis (that is, an optical axis of the objective optical system) O of the image pickup device 41c with respect to the objective optical system.

The angle around the center axis (that is, the optical axis of the objective optical system) O of the image pickup device 41c is set so that the two illumination ranges by the illuminating lights from the illuminating windows 22 and 23 are located in the vertical direction (UD direction) of the endoscopic image.

The range RR of the field of view of the image pickup device 41 c is set, that is, positioned, so that the two illumination ranges from the illuminating windows 22 and 23 are located in the vertical direction of the endoscopic image which is shorter than the lateral direction of the endoscopic image, and portions CC (shown by dotted-line circles) having the largest light amounts in the centers of the illuminating lights from the illuminating windows 22 and 23 at the time of proximity observation do not enter the range RR of the field of view. As a result, light distribution unevenness can be reduced at the time of proximity observation.

That is, the two illumination optical systems corresponding to the illuminating windows 22 and 23 are placed in the distal end portion 17, so that the two illumination ranges of the two illumination optical systems corresponding to the illuminating windows 22 and 23 are disposed along the direction of a shorter length of a length in the vertical direction and a length in the lateral direction of the endoscopic image which is obtained via the observation optical system. The two illumination optical systems corresponding to the illuminating windows 22 and 23 are placed in the distal end portion 17 so that the portions CC which are the brightest regions in the respective illumination ranges are located outside the endoscope image, at the time of enlarged observation.

Further, since the inclined plane portion DS1 is also formed between the air/water feeding nozzle 26 and the observation window 21, in a periphery of the observation window 21, a powerful water flows in such a manner as to be pushed out beyond the inclined plane portion DS1 and beyond the surface of the observation window 21 at the time of water feeding, but even if the water remaining in the air/water feeding conduit spouts from the air/water feeding nozzle 26, when water feeding is switched to air feeding, the portion DS 13 of the inclined plane portion DS1 becomes resistance so that water or a water drop does not reach the surface of the observation window 21, and functions as a resistance portion which causes water or a water drop to deviate without flowing toward the observation window 21 as shown by a chain double-dashed line.

As above, according to the aforementioned embodiment, the endoscope in which light distribution unevenness by the two illuminating lights which are respectively emitted from the two illuminating windows is reduced can be provided.

Note that although in the above explanation, as the example of the endoscope 2, an electronic endoscope is explained by being cited as an example, the present embodiment can be also applied to other endoscopes such as a fiber scope, and a rigid endoscope.

The present invention is not limited to the aforementioned embodiment, and various modifications, alterations and the like can be made within the range without departing from the gist of the present invention.

The present application claims benefit of Japanese Patent Application No. 2014-096290 filed in Japan on May 7, 2014, the disclosed contents of which are incorporated in the description, and the claims of the present application by reference.

**Claims**

1.  An endoscope, comprising:

    an observation optical system that is placed in a distal end portion of an insertion portion, and is capable of enlarged observation optically;
    a first illumination optical system that is placed in the distal end portion, has a first illumination lens in a distal end, and emits a first illuminating light;
    a second illumination optical system that is placed in the distal end portion, has a second illumination lens in a distal end, and emits a second illuminating light;

a first inclined plane portion that is provided between a peripheral edge portion of an objective lens in a distal end of the observation optical system and a peripheral edge portion of the first illumination lens;

a second inclined plane portion that is provided between a peripheral edge portion of the objective lens and a peripheral edge portion of the second illumination lens;

a first edge line portion that is formed at an intermediate portion of the first inclined plane portion, and is formed to protrude in a distal end direction of the insertion portion, between the peripheral edge portion of the objective lens and the peripheral edge portion of the first illumination lens; and

a second edge line portion that is formed at an intermediate portion of the second inclined plane portion, and is formed to protrude in the distal end direction of the insertion portion, between the peripheral edge portion of the objective lens and the peripheral edge portion of the second illumination lens,

wherein the observation optical system, the first illumination optical system and the second illumination optical system are placed so that relations of

$$H0 > HB1 > HL1, \text{ and } H0 > HB2 > HL2$$

are established, when a height to an incident surface of the objective lens of the observation optical system from a predetermined position in the distal end direction of the insertion portion is set as $H0$, a height to the first edge line portion from the predetermined position in the distal end direction of the insertion portion is set as $HB1$, a height to the second edge line portion from the predetermined position in the distal end direction of the insertion portion is set as $HB2$, a height to an exit surface of the first illumination optical system from the predetermined position in the distal end direction of the insertion portion is set as $HL1$, and a height to an exit surface of the second illumination optical system from the predetermined position in the distal end direction of the insertion portion is set as $HL2$, and the first illumination lens and the second illumination lens are placed to be point-symmetric or substantially point-symmetric with respect to a center axis of the objective lens of the observation optical system when the distal end portion is seen from the distal end direction, or to be line-symmetric or substantially line-symmetric with respect to a line passing through the center axis of the objective lens of the observation optical system.

2. The endoscope according to claim 1,
wherein the first illumination optical system and the second illumination optical system are placed in the distal end portion so that two illumination ranges of the first illumination optical system and the second illumination optical system are disposed along a direction of a shorter length of a length in a vertical direction and a length in a lateral direction of an endoscopic image which is obtained via the observation optical system.

3. The endoscope according to claim 1,
wherein a first angle formed by two inclined planes of the first inclined plane portion divided by the first edge line portion, and a second angle formed by two inclined planes of the second inclined plane portion divided by the second edge line portion are obtuse angles.

4. The endoscope according to claim 1,
wherein parts of the first and the second inclined plane portions are formed at a distal end cover member which is put on the distal end portion.

5. The endoscope according to claim 1,
wherein parts of the first and the second inclined plane portions are formed by an adhesive that connects and fixes a distal end cover member which is put on the distal end portion and the observation optical system.

6. The endoscope according to claim 1, comprising:

an air/water feeding nozzle placed in the distal end portion of the insertion portion;
a third inclined plane portion provided between the peripheral edge portion of the objective lens and the air/water feeding nozzle; and
a third edge line portion that is formed at an intermediate portion in the third inclined plane portion, and is formed to protrude in the distal end direction of the insertion portion, between the peripheral edge portion of the objective lens and the air/water feeding nozzle.

7.  The endoscope according to claim 6,
    wherein the first inclined plane portion, the second inclined plane portion and the third inclined plane portion are included in a continuous plane portion, and
    the continuous plane portion is formed to connect a first edge portion and a second edge portion of a distal end face of the distal end portion, and surround at least a part of the peripheral edge portion of the objective lens.

8.  The endoscope according to claim 1, further comprising:

    a third illumination optical system that is placed in the distal end portion, has a third illumination lens in a distal end, and emits a third illuminating light,
    wherein the third illumination optical system is more distant than the first and the second illumination optical system in a distance from the observation optical system, in the distal end portion, and is placed outside the endoscopic image.

# FIG. 1

# FIG. 2

# FIG. 3

# FIG. 4

# FIG. 5

# FIG. 6

# FIG. 7

# FIG. 8

# FIG. 9

# FIG. 10

**INTERNATIONAL SEARCH REPORT**

| International application No. |
|---|
| PCT/JP2015/059324 |

A. CLASSIFICATION OF SUBJECT MATTER
*A61B1/00*(2006.01)i, *G02B23/26*(2006.01)i

According to International Patent Classification (IPC) or to both national classification and IPC

B. FIELDS SEARCHED

Minimum documentation searched (classification system followed by classification symbols)
A61B1/00, G02B23/26

Documentation searched other than minimum documentation to the extent that such documents are included in the fields searched
Jitsuyo Shinan Koho          1922–1996   Jitsuyo Shinan Toroku Koho   1996–2015
Kokai Jitsuyo Shinan Koho    1971–2015   Toroku Jitsuyo Shinan Koho   1994–2015

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2012-179078 A  (Fujifilm Corp.),<br>20 September 2012 (20.09.2012),<br>paragraphs [0026] to [0029], [0045] to [0048];<br>fig. 9 to 12<br>& EP 2491848 A1         & CN 102648841 A | 1,3,4<br>2,5,6-8 |
| Y<br>A | JP 2007-202836 A  (Olympus Medical Systems Corp.),<br>16 August 2007 (16.08.2007),<br>paragraphs [0040], [0066]<br>(Family: none) | 1,3,4<br>2,5,6-8 |

☐  Further documents are listed in the continuation of Box C.        ☐  See patent family annex.

| | |
|---|---|
| * Special categories of cited documents:<br>"A" document defining the general state of the art which is not considered  to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>15 June 2015 (15.06.15) | Date of mailing of the international search report<br>23 June 2015 (23.06.15) |
|---|---|
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (July 2009)

**EP 3 092 940 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007289355 A

- JP 2014096290 A